# EUROPEAN PATENT APPLICATION

(11) **EP 1 369 690 A1**
(43) Date of publication of application: **10.12.2003**
(21) Application number: 02258113.6
(22) Date of filing: 26.11.2002
(51) Int. Cl.: G01N 33/543, G01N 33/68, G01N 33/74, G01N 33/566, G01N 33/94

(54) **Immobilised G-protein coupled receptors on an artificial lipid membrane of a liquid chromatography stationary phase and use thereof for screening**

(30) Priority: 05.06.2002 US 161705
(71) Applicant: RETT Corporation, Washington, DC 20008 (US)
(72) Inventor: Wainer, Irving, Washington, DC (US); Zhang, Yanxiao, Vancouver, BC, V6M 2G2 (CA); Beigi, Farideh, Baltimore, Maryland 21202 (US); Moaddel, Ruin, Germantown, Maryland 20874 (US)
(74) Representative: Paget, Hugh Charles Edward

(57) **Abstract**

This invention provides an immobilized G-protein coupled receptor (GPCR) on a support in a liquid chromatography system. The method of the invention provides means of evaluating the attachment of an agent to a GPCR comprising the steps of: (a) immobilizing a GPCR on an artificial membrane support in a column, (b) exposing the support with the GPCR to test the agent at vaiying concentrations in a liquid chromatography system, (c) eluting the test agent from the column, and (d) evaluating the elution profile of the test material from the column. Using this method, it is possible to evaluate the interaction of the test agent with the GPCR. Following elution. it is possible to directly determine molecular structure by passing the elute through other testing devices such as a mass spectrometer.

## Description

This application is related to:
US Patent Application No. 10/161,705 filed 05 June 2002;
US Patent Application No. 10/142,785 filed 13 May 2002;
US Patent Application No. 09/619,505 field 19 July 2000;
US Patent Application No. 09/255,881 filed 23 February 1999;
US Provisional Patent Application No. 60/075,745 filed 23 February 1998;
the contents of each of which is incorporated herein by reference in its entirety for all purposes.

### BACKGROUND OF THE INVENTION

### Field of the Invention

This invention relates to the immobilization of G-protein coupled transmembrane receptors on a support in a liquid chromatographic system, and to the uses thereof in methods of on-line screening. All references cited herein are incorporated by reference for all purposes.

### Background of the Technology

The combinatorial synthesis of chemical libraries has created an enormous pool of possible new drug candidates. Synthetic method capabilities, including phage display preparations, have outstripped the ability to determine corresponding biological activity. An initial step in the resolution of this problem has been the development of microtiter plates which contain immobilized receptors/antibodies. The use of these plates can rapidly reduce the number of possible candidates in a combinatorial pool from thousands to hundreds. However, assignment of relative activity within the reduced pool of compounds remains a slow and repetitive process.

The relationship between basic pharmacological processes and liquid chromatography (LC) studies have been emphasized by the inclusion of biomolecules as active components of chromatographic systems. A wide variety of immobilized biopolymer-based LC stationary phases (BP-SPs) have been developed using proteins, enzymes, cellulose and amylose, macrocyclic antibodies and liposomes. It has been demonstrated that the chromatographic retention and selectivity of BP-SPs are related to the properties of the non-immobilized biopolymer. For example, retention of a compound on an SP column containing immobilized human serum albumin has been used to evaluate the binding properties of the compounds to proteins.

The G-protein coupled receptor (GPCR) family is a broad-ranging collection of transmembrane receptors that play a vital role in biochemical and pharmacological processes. These receptors are key targets for new drug discovery and represent a significant share of the pharmaceutical market. The immobilized GPCR technology represents a rapid and straightforward approach to the development of new therapeutic agents for this family of receptors.

In addition, genomic studies of characterized GPCRs has led to the identification of a large number of GPCRs, but their function is often unknown. These receptors having no known function are otherwise known as orphan receptors. A key goal of the pharmaceutical industry is to identify agonists or antagonists for these orphan receptors and to elucidate their function as possible therapeutic targets.

The invention allows for immobilization of these GPCR orphan receptors, and rapid screening of a large number of compounds against these receptors. According to the invention, it is possible to identify active ligands that bind to these GPCR receptors, classify them in a particular GPCR family, and subsequently obtain first and later generation drugs for the treatment of illnesses associated with the particular GPCR, or its family.

The invention offers an improved alternative to the currently available high throughput screening methods that are based upon utilization of microtiter wells. In addition, on line screening is suitable for screening ligands from phage libraries in addition to those from chemical libraries:

G-protein coupled receptors (GPCRs) can be immobilized on silica-based chromatographic stationary phases and packed into glass or polymer tubes to produce liquid chromatography columns or immobilized directly on the surfaces of glass tubing. The resulting systems can be used in drug discovery and drug development programs by on-line determination of compounds that bind to the immobilized GPCR. These compounds can be isolated from chemical or biological mixtures that can be either simple or complex. Those compounds having a chemical process origin can be obtained utilizing combinatorial chemistry. Mixtures having a biological origin can be obtained using phage display. The system can also be used for the isolation, identification and characterization of antagonists and agonists to a GPCR.

The development of immobilized GPCR liquid chromatography columns represents a significant development relative to the previously reported receptor-based liquid chromatographic columns described in U.S. Patent Nos. 6,387,268 or 6,139,735. In the immobilization of the GPCR class of receptors, significant changes had to be made in the immobilization procedure to insure the successful production of the immobilized GPCR liquid chromatography stationary phases.

The methodology for the immobilization of a GPCR is illustrated using the production of an immobilized Opioid receptor column. However, the method is applicable to any member of the GPCR family.

### SUMMARY OF THE INVENTION

The invention provides an immobilized G-protein coupled receptor (GPCR) on a support in a liquid chromatography system. Using methods according to the invention, it is possible to immobilize a GPCR on the support, then expose the receptor to an agent that may bind to the GPCR. It is then possible to determine whether binding of the agent to the receptor has occurred.

It is also possible to expose the GPCR on the support to substances that might inhibit or promote interaction between the agent that is known to interact with the GPCR, then expose the support with the GPCR to the agent to determine whether or not the proposed inhibitor/promoter will, in fact, affect binding to the GPCR. Hence, using the means of the invention, it is possible to test the interaction of a potential drug utilizing the immobilized GPCR and evaluate possible candidates for inhibition or promotion of GPCR interactions.

### DETAILED INSCRIPTION OF THE INVENTION

It is the purpose of this invention to provide a means for the immobilization of a G-protein coupled membrane receptor in order to study ligand-receptor interactions utilizing Liquid Chromatography (LC) techniques. The fundamental processes of drug action, absorption, distribution and receptor activation; are all dynamic in nature and have much in common with the basic mechanisms involved in chromatographic distribution. The same basic intermolecular interactions (hydrophobic. electrostatic and hydrogen bonding) determine the behavior of chemical compounds in both a native biological and the artificial environment produced in liquid chromatography.

Although GPCRs play an important role in drug activity and are key targets in combinatorial chemistry screening tests, they have not been previously included in LC systems. This has been due, in part, to the disruption of the tertiary structure of the receptor produced by a covalent immobilization of a GPCR on a solid LC support. One solution to this problem is the immobilization of GPCR membrane receptors in the phospholipid layer of an immobilized artificial membrane (IAM) LC stationary phase.

A method according to the invention provides means of evaluating the binding of agents to receptors and comprises the steps of:
(a) immobilizing a GPCR receptor on an artificial membrane support in a column,
(b) exposing the support with the GPCR receptor to test an agent at varying concentrations in a liquid chromatography system,
(c) eluting the test agent from the column, and
(d) evaluating the elution profile of the test material from the column.

Using this method, it is possible to evaluate the interaction of a test agent with a GPCR membrane receptor. Following elution, it is possible to directly determine molecular content and structure by passing the elute through other analytical testing devices such as a mass spectrometer.

### EXAMPLES

The following examples are to provide one of ordinary skill in the art with a sufficient disclosure and description of how to carry out the invention and are not intended to limit the scope of what the inventors regard as their invention. Nor are they intended to represent or imply, that the experiments below are all, or the only experiments performed. Effort has been made to ensure accuracy with respect to the values to be used (e.g., amounts, temperatures, etc.), but some experimental error and deviation should be accounted for.

### EXAMPLE 1

Opiates are a large class of receptor-acting compounds that mediate various behaviors and effects, such as analgesia and euphoria. The Opioid family of GPCR receptors is characterized by the seven transmembrane helices coupled to the G-protein that mediate its down-regulation. Extensive studies have shown the existence of µ, δ and κ subtypes for these receptors. The main ligands for the subtypes are known to be endogenous peptides and hormones such as enkephalins, endorphins and dynorphins. The immobilization has been performed on a phospholipid monolayer of an immobilized artificial membrane (IAM™) chromatographic stationary phase.

In studying the Opioid GPCR receptors and their ligands, CHO-MOR, KOR, DOR and HEK-293-MOR cells were cultured, harvested and stored at -70°C. For the immobilization procedure, an approximately 50x10⁶ cell membrane pellet was homogenized in buffer for 5×10 seconds with a sonicator. For removal of large cell particles and organelles, the suspension was centrifuged at 180x g for 10 minutes. In order to separate the cell membrane the above supernatant was centrifuged at 55,000× g for 25 minutes. The resultant pellet was suspended in solubilization buffer containing 2% sodium cholate detergent over night at 4°C. The suspension was centrifuged at 100,000x g for 35 minutes.

To the supernatant now containing solubilized membrane proteins in detergent, 0.2 mg of IAM beads and 50 ul, ml or µl of phosphatidylcholine liposomes were added to get a total concentration of 70 µM. This mixture was stirred for about 3 hours at room temperature. Removal of detergent was accomplished by dialysis at 4°C in 2×1 L dialysis buffer for 2·3 days.

Upon removal of detergent the lipids and proteins reorganize and form a layer with the hydrocarbon chains of the IAM™ beads and are immobilized into the matrix. The IAM™ support containing fragments of the immobilized membrane and the receptor was centrifuged; the solid was collected and packed into a HR 5/2 chromatographic glass columns to obtain 0.4 ml of a gel bed. The column was connected to an HPLC pump and the ligand was injected separately and detected on-line by a radio-flow detector in both zonal and frontal chromatographic mode.

### EXAMPLE 2

Preliminary binding studies for some agonist and antagonist on µ, κ subtype show relative affinities to the receptor, In frontal chromatographic zone a break through in affinity studies for a κ agonist, U69593 showed an initial K_{d} = 84 nM for 147 pmole of binding sites. The K_{d} value for this compound is consistent with other reported values, K_{d}=1 nM.

A list of some of the well-known GPCRs and their related ligands is presented in Table 1.

**TABLE 1**

| **α and β adrenergic receptors** | **Non-peptide Ligand** |
|---|---|
| A1 and A2 adenosine receptors | Adenosine |
| c-AMP receptors | c-AMP |
| Histamine receptors | Dopamine |
| GABA-receptors | γ-aminobutyric acid |
| M1-M5 muscarinic receptors | Muscarinic |
| 5HT1 and 5HT2 receptors | Serotonine |

| | **Peptide Ligand** |
|---|---|
| ACTH-receptors | Adrenocorticotropic hormone |
| Opioid receptors | Opiate |
| TSH receptors | Thyroid stimulating hormone |
| GH receptors | Somastatin |
| Nmk, SK and SP receptors | Tachykinin |
| TSH receptors | Thyroid stimulating hormone |
| A2 receptors | Thromboxane |

| | **Other Stimuli** |
|---|---|
| Rhodopsin and cone-receptors | Light |
| B and T cell receptors | Immune recognition system |

Using methods of the invention, supports with the GPCR receptors may be exposed to compounds, then followed by chromatographic evaluation of the presence of the compound by chromatographic means to determine whether the compound is present on the support. Using one means of the invention, it would also be possible to determine whether proposed inhibitors or promoters of compound-receptor interaction will, in fact, inhibit or promote the interaction by exposing the support with GPCR receptors bound thereto to proposed candidate promoters or inhibitors, then to the compound, and followed by chromatographic evaluation of the support to determine whether the compound has been enhanced or inhibited from binding to the GPCR receptor by the candidate promoter or inhibitor under consideration.

While the invention has been exemplified using certain Opioid GPCR receptors, any GPCR receptor system may be used. Other GPCR receptors can be tested to determine if they function in a manner similar to those exemplified herein. Examples of other GBCR receptors are listed in Table 1 above,

Other supports than those exemplified which are HPLC-type supports known in the art may be used. Supports such as hydrogel beads or hydrophilic verticle support systems may be used in the methods of the invention. Because the methods of the invention require only evaluation of comparative elution volume profiles with the test material being fully eluted at the end of the study, the GPCR receptor binding column can be reused repeatedly.

The preceding specific embodiments are illustrative of the practice of the invention. This invention may be suitably practiced in the absence of any element or item not specifically described in this document. The complete disclosures of all patents, patent applications, and publications identified herein are incorporated into this document by reference as if individually incorporated in total.

Various modifications and alterations of this invention will become apparent to those skilled in the art without departing from the scope of this invention, and it should be understood that this invention is not to be unduly limited to illustrative embodiments set forth herein, but is to be controlled by the limitations set forth in the claims and any equivalents to those limitations.

## Claims

1. An artificial membrane support having at least one G-protein coupled receptor (GPCR) immobilized thereon, wherein said GPCR is immobilized such that its tertiary structure after immobilization permits binding to ligands that are bound by said at least one GPCR in vivo.

2. The artificial membrane support according to claim 1, wherein said GPCR is immobilized thereon utilizing a non-covalent bond attachment means with said artificial membrane support.

3. The artificial membrane support according to claim 1, wherein said GPCR is immobilized thereon utilizing a covalent bond attachment means with said artificial membrane support.

4. The artificial membrane support according to claim 1, wherein said GPCR is immobilized utilizing an immobilized artificial membrane (IAM) liquid chromatographic (LC) stationary phase comprising a phospholipid layer, said IAM LC stationary phase combined with said GPCR under conditions wherein the GPCR becomes immobilized in the phospholipid layer of said IAM- LC stationary phase such that the tertiary structure of the immobilized GPCR permits binding to said ligands that are bound by the GPCR in vivo.

5. The artificial membrane support according to claim 4, wherein said phospholipid layer is a monolayer.

6. The artificial membrane support of claim 1, wherein said GPCR is a member selected from the group consisting of:
A1 and A2 adenosine receptors; c-AMP receptors; Histamine receptors; GABA-receptors; M1 to M5 muscarinic receptors; 5HT1 and 5HT2 receptors; Adrenocorticotropic hormone receptors; Opioid receptors; Thyroid Stimulating Hormone receptors; GH receptors; Nmk SK and SP receptors; A2 receptors; Rhodopsin and cone-receptors; and B or T cell receptors.

7. The artificial membrane support of claim 6, wherein said GPCR is an Opioid receptor.

8. The artificial membrane support of claim 1, wherein said artificial membrane support is produced by the following steps:
obtaining an immobilized artificial membrane (IAM) liquid chromatographic (LC) stationary phase comprising a phospholipid layer; and
contacting said IAM LC stationary phase with said at least one GPCR under conditions wherein the GPCR becomes immobilized in the phospholipid layer of said IAM LC stationary phase such that the tertiary structure of the immobilized GPCR permits binding to said ligands that are bound by the GPCR in vivo.

9. A method of making an artificial membrane support comprising:
obtaining an immobilized artificial membrane (IAM) liquid chromatographic (LC) stationary phase comprising a phospholipid layer; and
contacting said IAM LC stationary phase with said GPCR under conditions wherein the GPCR becomes immobilized in the phospholipid layer of said IAM LC stationary phase.

10. The method of making according to claim 9, wherein the GPCR becomes immobilized in the phospholipid of said IAM LC stationary phase such that the tertiary structure of the immobilized GPCR permits binding to ligands that are bound by the GPCR in vivo.

11. A method of identifying whether a compound is a ligand, an agonist or an antagonist of at least one GPCR comprising (1) contacting an artificial membrane support having said GPCR immobilized thereon, to a liquid flow system comprising said compound, and (2) determining whether said compound becomes bound to said support.

12. The method according to claim 11, wherein said ligand, agonist or antagonist is obtained from a phage display library.

13. The method according to claim 11, wherein said ligand, agonist or antagonist is obtained from a combinatorial chemistry library,

14. The method according to claim 11, wherein said CPCR is immobilized such that its conformational structure after immobilization permits binding to ligands, agonists, or antagonists, that are bound by said GPCR when it is comprised in its native cellular environment, said artificial membrane support is contained in a liquid flow system; and said artificial membrane support is produced by the following steps:
(i) obtaining an immobilized artificial membrane (IAM) liquid chromatographic (LC) stationary phase comprising a phospholipid layer;
(ii) contacting said IAM LC stationary phase with at least one GPCR under conditions wherein that at least one GPCR becomes immobilized in the phospholipid layer of said IAM LC stationary phase such that the tertiary structure of said at least one immobilized GPCR permits binding to ligands, agonists or antagonists that are bound by said at least one GPCR when it is comprised in its native cellular environment; and
(iii) contacting said artificial membrane support comprising at least one immobilized GPCR to a liquid flow system.

15. The method of claim 11, which further comprises contacting said artificial membrane support to a liquid flow system containing a second compound that putatively affects binding of said first compound to said at least one GPCR; and determining whether said compound affects the binding of said first compound to said artificial membrane support.

16. A method for identifying, purifying or isolating a compound that affects the binding of a ligand to a GPCR that is bound by said compound comprising:
contacting an artificial membrane support having immobilized thereon at least one GPCR and to which the at least one GPCR is itself specifically bound to a ligand, to a liquid flow system that contains at least one compound that potentially affects the binding of said ligand which is bound to said immobilized GPCR.

17. The method according to claim 16, wherein said compound is obtained from a phage display library.

18. The method according to claim 16, wherein said compound is obtained from a combinatorial chemistry library.

19. The method according to claim 16, further comprising identifying, purifying or isolating said at least one compound based on its ability to affect the binding of said ligand to said immobilized GPCR.

20. The method according to claim 19, wherein said immobilized GPCR is immobilized to said artificial membrane support such that after immobilization the GPCR binds to ligands that are bound by said at least one GPCR when it is in vivo.

21. , The method according to claim 20, wherein said artificial membrane support is contained in a liquid flow system.

22. The method according to claim 21, wherein said artificial membrane support is produced by the following steps:
(I) obtaining an immobilized artificial membrane (IAM) liquid chromatographic (LC) stationary phase comprising a phospholipid layer;
(2) contacting said IAM LC stationary phase with at least one GPCR or GPCR-ligand complex under conditions wherein that at least one GPCR or GPCR-ligand complex becomes non-covalently immobilized in the phospholipid layer of said IAM LC stationary phase such that the immobilized GPCR permits binding to ligands that are bound by said GPCR when it is in vivo; and
(3) optionally contacting said artificial membrane support to which is immobilized the GPCR with at least one ligand that binds the immobilized GPCR under conditions that result in said ligand becoming bound to the GPCR immobilized to said support.

23. The method of claim 16, wherein said compound competes with said ligand for binding to said immobilized GPCR.

24. The method of claim 23, wherein said compound is obtained from a combinatorial chemical library,

25. The method of claim 23, wherein said compound is obtained from a phage display library

26. The method of claim 23, wherein said compound is contacted with said artificial membrane support comprising said immobilized GPCR/ligand complex by use of a liquid chromatographic system that comprises varying concentrations of said compound.

27. The method of claim 26, wherein the compound becomes bound to the support and the elution profile of said compound is evaluated upon eluting said compound from the support,
